# EUROPEAN PATENT SPECIFICATION

(11) **EP 0 716 856 B1**
(45) Date of publication and mention of the grant of the patent: **29.08.2001**
(21) Application number: 96101815.7
(22) Date of filing: 24.05.1989
(51) Int. Cl.: A61K 31/047, C07H 21/00

(54) **Therapeutic uses of 2',5'-oligoadenylate derivatives**
Therapeutische Anwendungen von 2',5'-Oligoadenylat-Derivaten
Utilisations thérapeutiques de dérivés du 2',5'-oligoadenylate

(30) Priority: 09.06.1988 US 204659
(43) Date of publication of application: 19.06.1996
(62) Divisional of application: 89906575.9
(73) Proprietor: Temple University of the Commonwealth System of Higher Education, Philadelphia PA 19122 (US)
(72) Inventor: Suhadolnik, Robert J., Roslyn, PA 19001 (US); Pfleiderer, Wolfgang, D-78464 Konstanz (DE)
(74) Representative: W.P. THOMPSON & CO.

(56) References cited:
- CARBOHYDRATE RESEARCH, vol. 216, 1991, pages 421-439, XP002000401 K. RUF: "Synthesis and properties of 5,6-dichlorobenzimidazole 2'-5'- and 3'-5'-nucleotide dimers and trimers"

## Description

### Field of the Invention

The invention relates to certain 2',5'-oligoadenylate analogs, their use in the preparation of medicaments and pharmaceutical compositions of such analogs.

### Background of the Invention

The full nomenclature of compounds mentioned in the present specification involves extremely long terms. It is customary for those skilled in the art to abbreviate these terms in a manner well known to them. These general and customary abbreviations are set forth herein below and may be utilized in the text of this specification.

### Abbreviations:

A, adenosine or adenylate or adenylyl
cordycepin or C or 3'-dA, 3'-deoxyadenosine(3'-deoxyadenylate)
ara-A, 9-β-D-arabinofuranosyladenine
EHNA, erthyro-9-(2-hydroxy-3-nonyl)adenine
A-3'-amino, 3'-amino-3'-deoxyadenosine
tubercidin, 4-amino-7(β-D-ribofuranosyl)pyrrolo-[2,3-d]pyrimidine
3'-dATP, 3'-deoxyadenosine triphosphate
ATP, adenosine triphosphate
I, inosine or inosinate or inosinylyl
Xylo-A or xyloadenosine, 9-β-D-xylofuranosylade-nine
dCF or 2'-deoxycoformycin, (R)-3-(2-deoxy-β-Derythropentofuranosyl)-3,6,7,8-tetrahydroimidazo[4,5-d][1,3]diazepine-8-ol
2-5A or 2',5'-oligo(A) or 2',5'-oligoadenylate, oligomer of adenylic acid with 2',5'-phosphodiester linkages and a triphosphate at the 5'-end
2',5'-cordycepin analog or 2',5'-oligocordycepin, oligomer of 3'-deoxyadenylic acid with 2',5'-phosphodiester linkages and a triphosphate at the 5'-end
2',5'-Aₙ or core oligomer, oligomer of adenylic acid with 2',5'-phosphodiester linkages
2',5'-A₃ or 2',5'-adenylate trimer core, adenylyl-(2',5')adenylyl(2',5')adenosine
2',5'-A₄ or 2',5'-adenylate tetramer core, adenylyl(2',5')adenylyl(2',5')adenylyl(2',5')adenosine
2',5'-3'dA₃ or 2',5'-C-C-C or 2',5'-cordycepin trimer core, 3'-deoxyadenylyl(2',5')3'-deoxyadenylyl-(2',5')3'-deoxyadenosine
2',5'-C-C-C-C or 2',5'-cordycepin tetramer core, 3'-deoxyadenylyl(2',5')3'-deoxyadenylyl(2',5')3'-deoxyadenylyl(2',5')3'-deoxyadenosine
3',5'-A₃, adenylyl(3',5')adenylyl(3',5')adenosine
2',5'-I₃ or 2',5'-inosine trimer core, inosinylyl-(2',5')inosinylyl(2',5')inosine
EBV, Epstein-Barr virus
EBNA, Epstein-Barr virus associated early nuclear antigen
HIV, human immunodeficiency virus, including HIV-1, HIV-2, and all other HIV subtypes
HBLV, human B-cell lymphotropic virus
HTLV, human T-cell leukemia virus, including HTLV-I, HTLV-II and HTLV-III, and all other HTLV sub-types
IFNα : α-interferon
rIFN-αA: recombinant α-interferon
dsRNA: double-strand ribonucleic acid
2',5'-A-A-Tu, adenylyl(2',5')adenylyl(2',5')tubercidin
2',5'-Tu-Tu-Tu, 2',5'-tubercidylyl(2',5')tubercidylyl(2',5')tubercidin
2',5'-A-A-ara-A, adenylyl(2',5')adenylyl(2',5')ara-A
2',5'-C-C-A, 3'-deoxyadenylyl(2',5')3'-deoxyadenylyl(2',5')adenosine
2',5'-A-C-C, adenylyl(2',5')3'-deoxyadenylyl-(2',5')3'-deoxyadenosine
2',5'-A-A-C, adenylyl(2',5')adenylyl(2',5')3'-deoxyadenosine
2',5'-C-A-C, 3'-deoxyadenylyl(2',5')adenylyl-(2',5')3'-deoxyadenosine
2',5'-C-C-A, 3'-deoxyadenylyl(2',5')3'-deoxyadenylyl (2',5') adenosine
2',5'-A-C-A, adenylyl(2',5')3'-deoxyadenylyl-(2',5')adenosine
2',5'-xylo-A₃, xyloadenylyl(2',5')xyloadenylyl-(2',5')xyloadenosine
2',5'-xylo-A₄, xyloadenylyl(2',5')xyloadenylyl-(2',5')xyloadenylyl(2',5')xyloadenosine
Ac, acetyl
Bz, benzyl
MMTr, 5'-0-p-methoxytrityl
2',5'-trityl-C₃, 5'-0-p-methoxytrityl-3'-deoxyadenylyl(2',5')3'-deoxyadenylyl(2',5')3'-deoxyadenosine
2',5'-trityl-A₃, 5'-0-p-methoxytrityladenylyl-(2',5')adenylyl(2',5')adenosine
2',5'-C-C-dCF, 3'-deoxyadenylyl(2',5')3'-deoxyadenylyl(2',5')2'-deoxycoformycin
2',5'-A-A-A-3'-amino, adenylyl(2',5')adenylyl-(2',5')3'-amino-3'-deoxyadenosine
SiTBD, t-butyldimethylsilyl or -Si(CH₃)₂C(CH₃)₃
2',5'-A_{(Si)}-A_{(Si)}-A, 3'-0-t-butyldimethylsilyladenylyl(2',5')3'-0-t-butyldimethylsilyladenylyl(2',5')-adenosine
2',5'-A-A-A-3'-0-methyl, adenylyl(2',5')adenylyl-(2',5')3'-0-methyladenosine
2',5'-A-A-A-3'-0-pentyl, adenylyl(2',5')adenylyl-(2',5')3'-0-pentyladenosine
2',5'-A-A-A-3'-0-hexyl, adenylyl(2',5')adenylyl-(2',5')3'-0-hexyladenosine
2',5'-A-A-A-3'-0-heptyl, adenylyl(2',5')adenylyl-(2',5')3'-0-heptyladenosine
2',5'-EHNA-A-A, erythro-9-(2-hydroxy-3-nonyl)-adenylyl(2',5')adenylyl(2',5')adenosine

The abbreviation for the "tetramer" compounds comprising the adenylyl (A) and 3'deoxyadenylyl (C) moieties is illustrated by the following:
2',5'-A-A-C-C, adenylyl (2',5')adenylyl (2',5')3'-deoxyadenylyl(2',5')3'-deoxyadenosine

With the expansion of the knowledge of the antiviral state induced by interferon, attention has been focused .on the chemical and enzymatic synthesis and biological properties of the 2',5'-oligoadenylates as mediators of the antiviral response. 2',5'-Oligo(A) is a component of a natural, broad-spectrum antiviral defense mechanism in plants and animals. The 2-5A pathway, also known as the 2-5A/RNase L pathway or antiviral system, is widely accepted to be involved in the antiviral mechanism of interferon, and may also be involved in the regulation of cell growth and differentiation. According to that pathway, 2-5A is synthesized from ATP by 2',5'-oligoadenylate synthetase [ATP: (2'-5')oligo(A)-adenyltransferase (EC 2.7.7.19)], hereinafter "2-5A synthetase". The enzyme is activated by dsRNA. 2-5A exerts its biological effects by binding to and activating its only known target enzyme, the unique 2-5A dependent endoribonuclease RNase L. The latter cleaves viral and cellular mRNA or rRNA, thereby inhibiting protein synthesis. Hovanessian et al., Eur. J. Biochem. 93:515-526 (1979); Kerr et al., Proc. Natl. Acad. Sci. USA 75:256-260 (1978). However, the short half-life of the authentic 2-5A molecule in biological systems is an acknowledged disadvantage in the control of viral replication.

"Human B-lymphotropic virus", also known as "human B-cell lymphotropic virus" (HBLV), which is characterized by a large molecular weight double-stranded DNA genome is morphologically similar to viruses of the herpes virus family, but is readily distinguishable from the known human and non-human primate herpes viruses by host range, in vitro biological effects, antigenic features and genome. Salahuddin et al., Science 234:596-601 (1986); Josephs et al., Science 234:601-602 (1986). The virus has been observed to selectively infect freshly isolated human B-cells, which are converted into large, refractile mono- or binucleated cells with nuclear and cytoplasmic inclusion bodies. HBLV is suspected to be the cause of a chronic mononucleosis-like syndrome characterized by chronic fatigue lasting more than a year.

Human immunodeficiency virus ("HIV"), also known as human T-cell leukemia virus III ("HTLV-III"), the etiologic agent of acquired immune deficiency syndrome, is a type D retrovirus. As in all retroviruses, an essential feature of HIV replication is reverse transcription of the plus-strand RNA genome into DNA, a process which requires an RNA dependent DNA polymerase, reverse transcriptase. This enzyme is viral-encoded and is found associated with genomic RNA in mature HIV virions. The exclusiveness of reverse transcriptase to retroviruses and viruses requiring a short reverse transcription step makes reverse transcriptase a major target for antiviral, and particularly for antiretroviral, therapeutic intervention.

What is needed is a method for controlling HIV, chronic fatigue caused by HBLV, and other disease states characterized by a 2-5A pathway defect using compounds that are more metabolically stable and active than authentic 2-5A.

### Summary of the Invention

In accordance with the invention, 5,6-dichlorobenzimidazylyl(2,'5')5,6-dichlorobenzimidazylyl(2',5')5,6-dichlorobenzimidazole riboside, or the 5' mono-, di- and triphosphate thereof, or a pharmaceutically acceptable salt of any of them (hereinafter "5,6-dichlorobenzimidazyl 2-5A analogs") is provided.

The invention is further directed to pharmaceutical compositions of the compounds, the compounds for use in medicine and their use for the preparation of medicaments for treating retroviral infections.

### Detailed Description of the Invention

Administration of the 5,6-dichlorobenzimidazyl 2-5A analogs will render increased protection against disorders characterized by a 2-5A defect, particularly protection against retroviral infection in animals and humans. By "2-5A defect" as used herein is meant any manifestation, condition or interruption of the 2-5A pathway which results in a decrease in the production of authentic 2-5A, and/or the interruption of 2-5A-dependent activation of RNase L. Afflictions characterized by a 2-5A defect include, for example, the following: retroviral infection, particularly HTLV infection, most particularly HIV infection, chronic fatigue, and cutaneous T-cell lymphoma; chronic myelogenous leukemia; acute leukemia; cancer; T-cell leukemia; Alzheimer's disease; Parkinson's disease; multiple sclerosis; autoimmune disease; and surgery- and other trauma-induced immune dysfunction.

The defect is apparent in diseases, such as the above disorders caused by chronic viral infection, immune cell defects or both. 2-5A pathway defects are particularly manifested in diseases characterized by both chronic viral infection and immune-cell defects.

Structural modification of the 2-5A molecule at the 3'-hydroxyl groups and elsewhere provides 2-5A analogues with remarkably increased metabolic stability to 2'-phosphodiesterases and cellular nucleuses, while maintaining the ability to activate RNase L. Likewise modification of native 2-5A by substitution of the terminal nucleotide results in a more stable molecule. Persistent, high intracellular concentration of the metabolically stable 5,6-dichlorobenzimidazyl 2-5A analogs are a consequence of their increased stability.

The longer-lasting pharmacological activity of the 5,6-dichlorobenzimidazyl 2-5A analogs offer a more favorable therapeutic ratio. This allows a decreased frequency of administration relative to 2-5A, which is metabolicly unstable. Decreased frequency of administration is important due to the chronic nature of many afflictions characterized by 2-5A pathway defects.

The 5,6-dichlorobenzimidazyl 2-5A analogs are particularly useful in the treatment of infections caused by retroviruses. The 2-5A pathway defect associated with retroviral infection comprises the inactivation of the pathway caused by the virus' interference with the activation of 2-5A synthetase by dsRNA. In the absence of 2-5A synthetase activation, 2-5A production, and hence activation of RNase L, is reduced. According to the present invention, exogenous, metabolically stable 5,6-dichlorobenzimidazyl 2-5A analog is administered to counteract this retrovirally-caused defect in the 2-5A pathway. These 2-5A analogs, like authentic 2-5A, are capable of activating RNase L, which cleaves viral RNA.

The 5,6-dichlorobenzimidazyl 2-5A analogs are particularly useful in protecting against infection by the various human T-cell leukemia viruses (collectively "HTLV"), such as HTLV-I, which cause cutaneous T-cell lymphoma; HTLV-II, which causes Sezany lymphoma: HTLV-III; and HTLV-IV, which is presently believed to be the etiologic agent of multiple sclerosis. Each of the HTLV viruses is a retrovirus. Also known as "HIV-1", HTLV-III is responsible for causing acquired immune deficiency syndrome ("AIDS"). The compounds are further believed useful in treating HIV-2, a second serologically distinct HIV subtype. Hereinafter (HIV) shall mean either HIV-1 or HIV-2, and any other HIV subtypes now or hereinafter known.

HTLV-infected patients, in particular HIV-1-infected patients, have been shown to demonstrate unusually low levels of 2-5A and/or RNase L activity in blood mononuclear cells. Blood mononuclear cells from healthy individuals, by contrast, display higher 2-5A levels, on average, and RNase L activity is readily detectable. Likewise blood mononuclear cells of chronic fatigue-inflicted individuals exhibit low 2-5A levels, and evidence the appearance of novel RNA cleavage products, distinct from the specific cleavage products observed in blood mononuclear cells from normal individuals.

While the practice of the invention is illustrated herein with regard to the treatment of HIV-1 infection, which is generally regarded as a prototypical retrovirus, the invention has application to the treatment of any diseases wherein the etiologic agent comprises a retrovirus. Additional retrovirus which infect man include, for example, the various non-HIV HTLV virus, discussed above.

The various afflictions characterized by a 2-5A pathway defect, in particular retroviral infection, most particularly HIV infection, may therefore be treated by the administration of exogenous, metabolically stable analogs of 2-5A to counteract the 2-5A system defect with the disease state.

For pharmaceutical use, the 5,6-dichlorobenzimidazyl 2',5'-oligoadenylate analogs may be taken up in pharmaceutically acceptable carriers. Such carriers for preparation of pharmaceutical compositions of the invention may be either organic or inorganic, solid or liquid in nature. Suitable solid carriers include gelatin, microcrystalline cellulose, lactose, starches, and magnesium stearate. Suitable liquid carriers include water and alcohols such as ethanol, benzyl alcohol and poly(ethylene glycols). The preferred liquid carriers for injectable preparations include water, saline solution, dextrose solution and glycol solutions such as aqueous propylene glycol or aqueous poly(ethylene glycol). The properties of the formulations may be enhanced by the addition of one or more adjuvants possessing properties as viscosity enhancers, surfactants, pH modifiers, preservatives, sweeteners, stability enhancers, coloring agents, suspending agents, granulating agents, coating agents, disintegration modifiers, propellants, emulsifying agents and hymectants. The resulting compositions may be in the form of solutions, suspensions, tablets, capsules, ointments, elixirs, injectable compositions and the like.

The dosage administered depends upon the severity of the infection or affliction and the size and weight of the subject. The dosage may vary over a wide range depending on the nature of the affliction, and the size and weight of the subject. According to one embodiment, the compounds are prepared as a solution of 0.1-100 mg/ml in water, phosphate buffered saline or other appropriate fluid, or may be prepared as a tablet containing 0.01-1 gram active compound.

The compounds may be administered in the form of water-soluble salts. Pharmaceutically acceptable water-soluble salts include, for example, the sodium, potassium and ammonium salts of the active compounds. They are readily dissolved in water or saline solution. The formulation may contain additional agents, such as a sugar or protein, to maintain the osmotic balance.

The 5,6-dichlorobenzimidazyl 2',5'-oligoadenylate analogs may be administered in doses of about 0.1 mg to about 1 gram to animals or humans afflicted by, or suspected of affliction by, or at risk of affliction by, any of the various conditions characterized by a 2-5A pathway defect. The total daily dosage may vary, for example, from about 0.001 gram to about 1 gram, although lower or higher amounts may be administered. A preferred daily dose is from about 0.01 gram to about 0.1 g of active ingredient. The compounds may be administered by any of several routes, including, but not limited to, intravenous injection, intraperitoneal or intramuscular injection, and oral administration. Techniques for accomplishing such administration are routine and known in the medical art. Administration may be as frequent as several times a day or as infrequent as weekly. For intravenous injection, particularly for treatment of HIV, a solution containing about 0.1 to about 1.0 milligram per ml of active ingredient is preferred.

It is also contemplated that the 5,6-dichlorobenzimidazyl 2',5'-oligoadenylate analogs may be administered topically to treat skin lesions associated with any of the disease states characterized 2-5A pathway defect. A sufficient amount of a preparation containing one or more of the analogs may be applied to cover the lesion or affected area. An effective concentration of active agent is from about 10⁻³ M to about 10⁻⁵ M, with about 10⁻⁴ M being preferred.

In addition to administration with conventional carriers, the 5,6-dichlorobenzimidazyl 2-5A analogs may be administered by a variety of specialized oligonucleotide or nucleic acid delivery techniques, such as by encapsulation in unilameller liposomes or reconstituted sendai virus envelopes, or by conjugation to carrier molecules such as poly(L-lysine). Such methods are disclosed in International Patent Application WO 89/03683.

The 5,6-dichlorobenzimidazyl 2',5'-oligoadenylate analogs may be chemically synthesized from 5,6-dichlorobenzimidazole riboside according to the following general protection, condensation and deprotection methods, as appropriate. A blocked adenosine-2'-phosphodiester is prepared by blocking the 6-amino position with benzoyl, blocking the 5' position with p-methoxytrityl and optionally blocking the 3' position with t-butyldimethylsilyl. A blocked nucleoside is prepared by blocking the 2' and 3' positions with acetyl, benzoyl or t-butyldimethylsilyl. Preparation of suitably blocked adenosine-2'-phosphodiesters is carried out by adding the t-butyldimethylsilyl group to the 3'-0-t-butyldimethylsilyl-isomeric-3'-hydroxyl group of N⁶-benzoyl-5'-0-(4-methoxytrityl)adenosine by condensing the latter compound with t-butyldimethylsilylchloride using imidazole in pyridine to yield the 3'-0-t-butyldimethylsilyl derivative. Phosphorylation of the derivative is carried out using 2,5-dichlorophenyl-phosphorotriazolide in pyridine to yield a suitably blocked adenosine-2'-phosphodiester. This preparation is described in R. Charubala, E. Uhlmann, and W. Pfleiderer, Liebigs Ann. Chem., 2392 (1981) and R. Charubala, W. Pfleiderer, Tetrahedron Lett. 21, 4077 (1980). The blocked adenosine-2'-phosphodiester is condensed with the blocked nucleoside in the presence of a condensing reagent which causes blocking of the phosphate functions to form a fully protected dinucleosidemonophosphotriester.

The resulting fully protected condensate is then detritylated at the terminal 5' position with a detritylating agent and condensed with a further adenosine-2'-phosphodiester, blocked as described above, to form a fully protected 2',5'-trinucleosidediphosphoditriester, or 2',5' trimer core. The fully protected trimer core is then treated with appropriate deprotecting reagents to achieve complete deprotection and conversion to 2',5' trimer core.

5,6-dichlorobenzimidazylyl-(2,'5')5,6-dichlorobenzimidazylyl-(2',5')5,6-dichlorobenzimidazole riboside is prepared by condensing commercially available 5,6-dichlorobenzimidazole riboside (Sigma, Cat. No. D5893) according to methods described herein.

The preparation of 2',5'-oligoadenylate analogs is illustrated in the following non-limiting examples, although the specific compounds prepared fall outside the scope of the present invention.

### Example 1

### Preparation of Structurally Modified 2',5'-Adenylate Trimer Cores

Structurally modified trimer core analogs of 2',5'-adenylate may generally be prepared as follows, although the specific compounds prepared in this example fall outside the present invention.

One mmole of suitably blocked adenosine-2'-phosphodiester having the general formula of reactant (I) was prepared from adenosine or cordycepin according to the method of Charubala, R., Uhlmann, E., and Pfleiderer, W., Liebigs Ann. Chem., 2392 (1981) and Charubala, R., and Pfleiderer, W., Tetrahedron Lett. 21, 4077 (1980). Examples of suitably blocked adenosine-2'-phosphodiesters are represented by compounds 1 and 2, listed in Table 1.

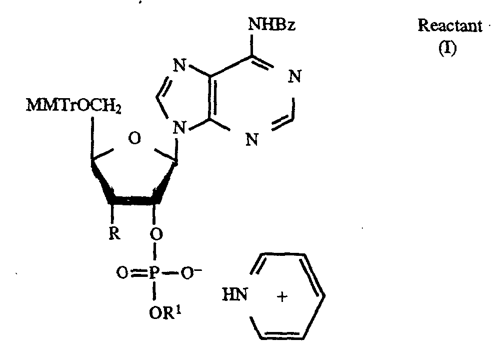

**TABLE 1**

| Compound Name | Compound No. | | |
|---|---|---|---|
| | | R | R¹ |
| Pyridinium N⁶ benzoyl-3'-0-t-butyldimethylsilyl-5'-0-p-methoxytrityladenosine-2-(2-chlorophenyl)-phosphate | 1 | OSiTBD | 2-chlorophenyl |
| Pyridium N⁶-benzoyl-5'-0-p-methoxytrityl-3'-deoxyadenosine-2'-(2-chlorophenyl)-phosphate | 2 | H | 2-chlorophenyl |

Reactant (I) was combined with a blocked nucleoside having the general formula of reactant (II) below. Reactant (II) is exemplified by compounds 3 - 9 listed in Table 2.

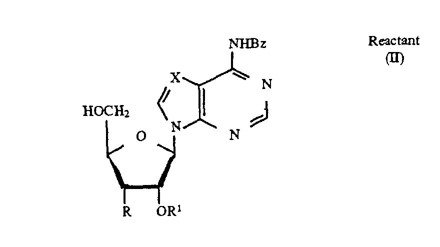

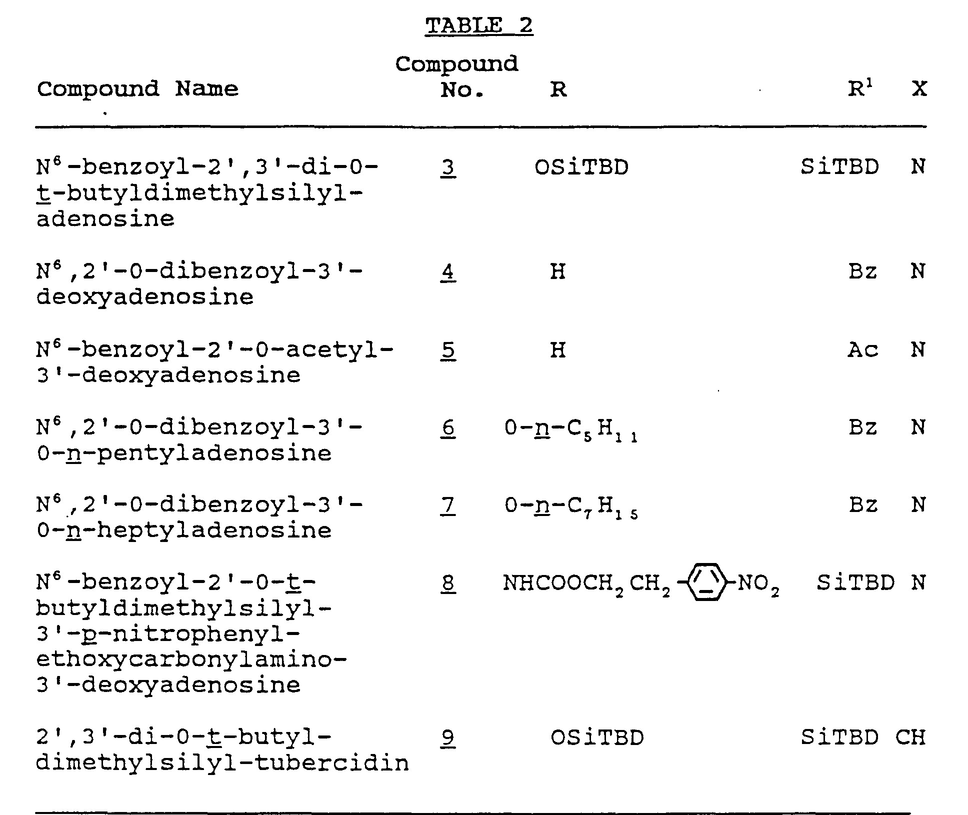

Compounds 3 and 9 are prepared treating N⁶-benzoylated adenosine with monomethoxytrityl chloride in pyridine to yield the 5'-monomethoxytrityl derivative. The derivative is treated with t-butyldimethylsilylchloride in a mixture of pyridine and methylimidazole to form the N⁶-benzoyl-2',3'-di-0-t-butyldimethylsilyl-5'-monomethoxytrityladenosine. The trityl group is removed by acetic acid to produce compound 3. Compound 9 is synthesized analogously, starting with N⁶-benzoylated tubercidin. This method of preparation is a described in Charubala, et al., Liebigs Ann. Chem. 2392 (1981).

Compound 4 is prepared by treating N⁶-benzoylated-3'-deoxyadenosine with monomethoxytritylchloride to yield N⁶-benzoyl-5'-0-monomethoxytrityl-3'-deoxyadenosine, which is converted to N⁶-2'-0-dibenzoyl-3'-deoxyadenosine by benzoylation of the 2'-hydroxyl group with benzoylchloride followed by detritylation with 80% acetic acid for 30 minutes. This method of preparation is as described in Tetrahedron Lett. 21, 4077 (1980).

Compound 5 is prepared by converting N⁶-benzoyladenosine with t-butyldiphenylchlorosilane to the 5'-silylated nucleoside in pyridine. The 5'-silylated nucleoside is treated with triethyl orthoacetate followed by boron trifluoride/diethyl ether and sodium iodide in CH₃CN (0°C, 1 hour) to yield the 3'-iodoacetyl derivative. The iodoacetyl derivative is converted to compound 5 by treatment with tributyltinhydride in toluene (80°C, 1 hour) and desilylation with ammonium tetrabutyl fluoride in tetrahydrofuran. This method of preparation is as described by Engles, J., in Tetrahedron Lett. 21, 4339 (1980).

Compounds 6 and 7 are prepared by treating N⁶-benzoyl adenosine with tritylchloride in pyridine and refluxing for 2 hours. N⁶-benzoyl-5'-trityladenosine is isolated by extraction with chloroform. The water from the chloroform phase is removed by drying with sodium sulfate. N⁶-benzoyl-2',5'-di-0-trityladenosine and N⁶-benzoyl-3',5'-di-0-trityladenosine are isolated by preparative silica gel thin layer chromatography in chloroform/ethanol. Compounds 6 and 7 are prepared by treating the isolated compounds with n-pentylchloride and n-heptylchloride, respectively, under reflux in a suspension of sodium hydroxide in benzene. The solution is neutralized by refluxing in acetic acid followed by the addition of diethyl ether and water. The reaction products are extracted with chloroform followed by thin layer chromatography with chloroform: methanol (4:1) on silica gel plates. The trityl and benzoyl groups are removed by refluxing in acetic acid for one hour, cooling, extraction with diethyl ether, followed by concentration and cooling to yield crystalline compounds 6 and 7. This method of preparation is carried out according to Blank, H.U., Franne, D., Myles, A. and Pfleiderer, W., Justus Liebigs Ann. Chem. 742, 34 (1970).

Compound 8 was prepared as follows. 1 mmole of 3'-amino-3'-deoxyadenosine was reacted with 1.2 mmole of 1-methyl-3-nitrophenylethoxy carboxylimidazolium chloride in dimethylformamide, followed by the addition of hexamethyldisilazane to block the 2',5' and 6-amino positions. 1.1 mmole of benzoyl chloride in pyridine was added at room temperature to produce blocked N⁶-benzoyl-2',5'-disilyladenosine. The reaction mixture was poured into methanol-NH₃ to remove the 2' and 5' silyl groups. Reaction with MMTr chloride in pyridine yielded the 5'-MMTr derivative. Tert-butyldimethylsilyl chloride in a mixture of pyridine and 1-methylimidazole was then added to the 5'-MMTr derivative and 5'-detritylated as in Example 1 to form compound 8.

Reactants (I) and (II) were combined to produce the intermediate having the general formula of dimer (III) as follows. 0.95 Mmole of reactant (II) and condensing reagents 2,4,6-triisopropylbenzenesulfonyl chloride (2 mmole) and 1-methylimidazole (6 mmole) were combined and stirred for 1 h at room temperature. The reaction is stopped by adding 30 ml of aqueous phosphate buffer pH 7 and extracted with 150 ml of chloroform. The chloroform layer was washed twice with 50 ml of water, dried over sodium sulfate about 1-2 h and filtered. The chloroform was evaporated to a small volume and then applied to a silica gel column (20 x 2.5 cm) for purification. Chromatography was performed first with chloroform and then with chloroform/methanol (99/1, v/v) to elute the fully protected dinucleosidemonophosphotriester produce of the general formula of dimer (III). Evaporation gave a solid foam of dimer (III) exemplified by compounds 10-17 (Table 3).

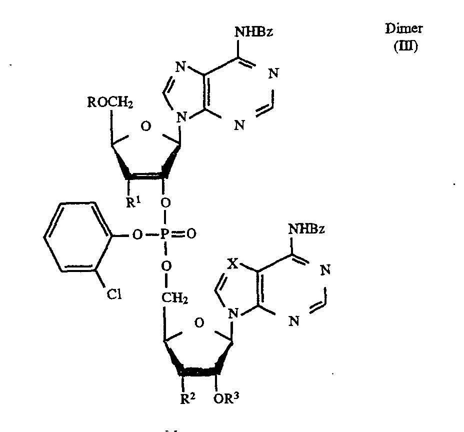

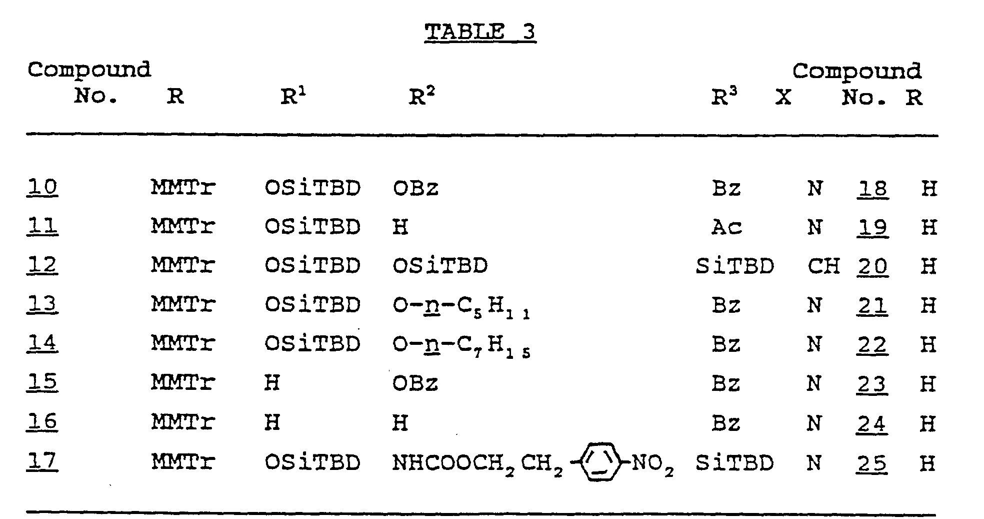

One mmole of the fully protected dimer (III) was stirred at room temperature for 30 minutes in 20 ml of 2% p-toluenesulfonic acid in dichloromethane/methanol (4/1, v/v) for detritylation. 20 ml of phosphate buffer pH7 was added and subsequently extracted several times with 200 ml of dichloromethane. The organic phase was washed with water, dried over sodium sulfate, evaporated to a small volume and then applied to a silica gel column (20 x 2.5 cm) for purification. Elution was performed with chloroform (400 ml) followed by chloroform/methanol (98/2, v/v/). Evaporation of the main fraction gave a 80-90% yield of the detritylated dinucleosidemonophosphotriester (dimer (III)), exemplified by compounds 18-25, (Table 3).

The fully protected 2',5'-trinucleosidediphosphoditriester, having the general formula of trimer (IV) and exemplified by compounds 26 - 34 (Table 4, below), was prepared from the 5'-detritylated dimer (III) as follows.

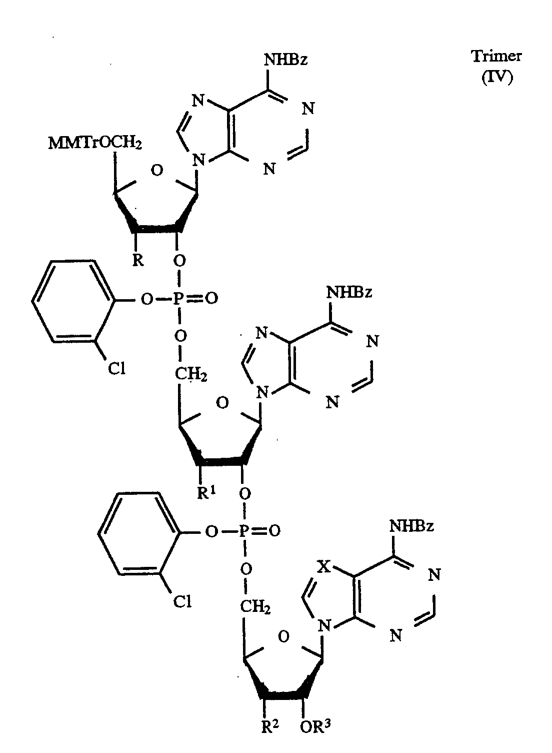

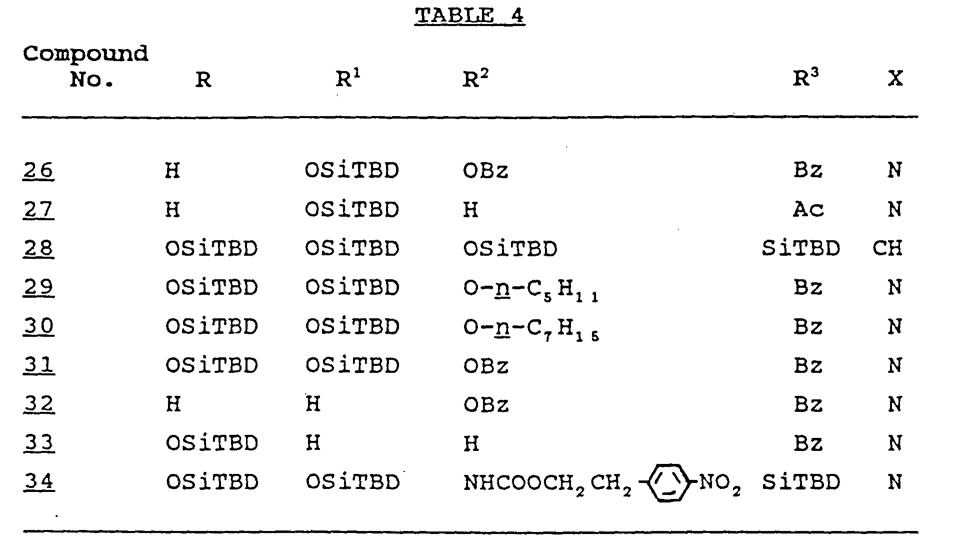

1.05 mmole of the starting adenosine-2'-phosphodiester (reactant (I)), was condensed with 1 mmole of the 5'-detritylated dimmer (III) (compounds 18 - 25) in 10 ml of absolute pyridine using 3 mmole of 2,4,6-triisopropylbenzenesulfonyl chloride and 9 mmole of 1-methylimidazole as condensing agents. Work-up was performed after 2 hrs in the manner as described above. Quenching with phosphate buffer, followed by extraction with dichloromethane and silica gel chromatography in chloroform and chloroform/ methanol (99/1/ to 98/2, v/v) yielded 70-90% of fully protected trimer (IV) (compounds 26 - 34), as a chromatographically pure amorphous powder.

The fully protected 2'-5'-trinucleosidediphosphoditriester, trimer (IV), was deprotected to trimer core (V) as follows.

0.01 mmole of trimer (IV) was treated with a solution of 0.073 g p-nitrobenzaldoxime and 0.07 g tetramethylguanidine in 2 ml of dioxane water (1/1, v/v) for 16 hrs at room temperature to deblock the o-chlorophenyl group. After evaporation to dryness and coevaporation four times with water, 20 ml of concentrated ammonium hydroxide was added and the solution stirred for 2 days at room temperature to deprotect the acyl groups. The solution was then evaporated again, and the residue was dissolved in 25 ml of water and washed four times with 10 ml of chloroform each time. The water layer was evaporated to dryness and coevaporated ten times with 10 ml absolute pyridine each time. The residue was then treated with 2 ml of an 0.5 M solution of anhydrous tetrabutylammonium fluoride in absolute pyridine for 16 hrs to remove the t-butyldimethylsilyl groups. After evaporation, treatment of the residue with 5 ml of 80% acetic acid for 6 hrs at room temperature lead to cleavage of the p-methoxytrityl group. The solution was again evaporated, the residue dissolved in 15 ml of water, and extracted four times with 5 ml of chloroform each time. The aqueous layer was evaporated and then coevaporated several times with water until the smell of acetic acid disappeared. The residue was dissolved in 10 ml of water and applied to a DEAE-Sephadex A-25 column (60 x 1 cm) for ion-exchange chromatography with a gradient of 0.001 - 0.5 M triethylammonium bicarbonate. The main fraction was evaporated, then coevaporated several times with water. Trimer core (V), a fully deprotected 2',5'-trinucleosidediphosphate, was isolated by lyophilization of the aqueous solution to give 70-90% of an amorphous solid. Trimer core (V) is exemplified by compounds 35 - 45 (Table 5).

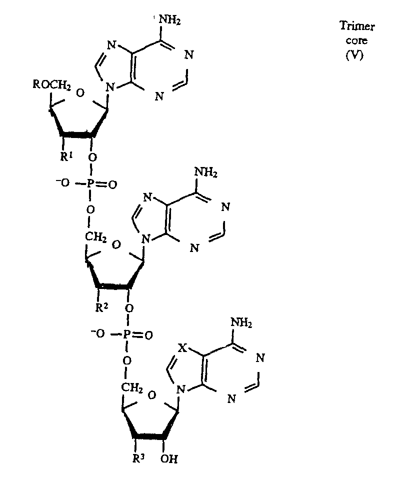

**TABLE 5**

| Compound Name | Compound No. | | | | | |
|---|---|---|---|---|---|---|
| | | R | R¹ | R² | R³ | X |
| 2',5'-A-A-C | 35 | H | OH | OH | H | N |
| 2',5'-C-A-C | 36 | H | H | OH | H | N |
| 2',5'-A-A-Tu | 37 | H | OH | OH | OH | CH |
| 2',5'-A-A-A-3'-0-pentyl | 38 | H | OH | OH | O-n-C₅ H₁₁ | N |
| 2',5'-A-A-A-3'-0-heptyl | 39 | H | OH | OH | O-n-C₇ H₁₅ | N |
| 2',5'-A_{(Si)}-A_{(Si)}-A | 40 | H | OSiTBD | OSiTBD | OH | N |
| 2',5'-C-C-A | 41 | H | H | H | OH | N |
| 2',5'-A-C-C | 42 | H | OH | H | H | N |
| 2',5'-A-A-3'-amino | 43 | H | OH | OH | NH₂ | N |
| 2',5'-Trityl-A₃ | 44 | MMTr | OH | OH | OH | N |
| 2',5'-Trityl-C₃ | 45 | MMTr | H | H | H | N |
| 2',5'-C-C-C-5'-monophosphate | 46 | H₂O₃P | H | H | H | N |

### Example 2

### Preparation of 3'-0-t-butyldimethylsilyladenylyl (2',5')3'-0-t-butyldimethylsilyladenylyl(2',5') adenosine

0.01 mmole of N⁶-benzoyl-3'-0-t-butyldimethylsilyl-5'-0-p-methoxytrityladenylyl(2'-o-chlorophenyl-5')N⁶-benzoyl-3'-0-t-butyldimethylsilyladenylyl(2'-o-chlorophenyl-5')N⁶, 2'-0,3'-0-tribenzoyladenosine (compound 31, Table 4) was treated according to the procedure of Example 1, except that the deprotection step of treatment with tetrabutylammonium fluoride was omitted, in order to produce 2',5'-A_{(Si)}-A_{(Si)}-A (compound 40, Table 5).

### Example 3

### Preparation of adenylyl(2',5')adenylyl (2',5')3'-amino-3'-deoxyadenosine

0.01 mmole of N⁶-benzoyl-3'-0-t-butyldimethylsilyl-5'-0-p-methoxytrityladenylyl(2'-o-chlorophenyl-5')N⁶-benzoyl-3'-0-t-butyldimethylsilyladenylyl(2'-o-chlorophenyl-5')N⁶-benzoyl- 2'-0-t-butyldimethylsilyl-3'-p-nitrophenylethoxycarbonylamino-3'-deoxyadenosine (compound 34, Table 4) was treated according to the deprotection procedure of Example 1 wherein the p-nitrophenylethoxycarbonyl group was cleaved simultaneously with the silyl groups by tetrabutylammonium fluoride in a β-elimination process. Subsequent decarboxylation yields 2',5'-A-A-3'-amino (compound 43, Table 5).

### Example 4

### Preparation of 5'0-p-methoxytrityladenylyl (2',5')adenylyl(2',5')adenosine

0.01 mmole of N⁶-benzoyl-3'-0-t-butyldimethylsilyl-5'-0-p-methoxytrityladenylyl(2'-o-chlorophenyl-5')N⁶-benzoyl-3'-0-t-butyldimethylsilyladenylyl(2'-o-chlorophenyl-5')N⁶,N⁶-2'-0,3'-0-tetrabenzoyladenosine prepared according to the method of Charubala, R., Uhlmann, E., and Pfleiderer, W., Liebigs Ann. Chem., 2392 (1981) was treated according to the deprotection procedure of Example 1 except that the last step of acetic acid treatment was omitted. The product, 2',5'-trityl-A₃ (compound 44, Table 5) was isolated, purified by DEAE-Sephadex A-25 chromatography, and lyophilized to form the amorphous pure compound 44 as a powder in 85% yield.

### Example 5

### Preparation of 5'-0-p-methoxytrityl-3'-deoxyadenylyl (2',5')3'-deoxyadenylyl(2',5')3'-deoxyadenosine

0.01 mmole of N⁶-benzoyl-5'-0-p-methoxytrityl-3'-deoxyadenylyl(2'-o-chlorophenyl-5')N⁶-benzoyl-3'-deoxyadenylyl(2'-o-chlorophenyl-5')N⁶,N⁶,2'-0-tribenzoyl-3'-deoxyadenosine prepared according to the method of Charubala, R., and Pfleiderer, W., Tetrahedron Lett. 21, 4077 (1980) was treated according to the deprotection procedure of Example 1 except that the steps of treatment with tetrabutylammonium fluoride and acetic acid were omitted. The product, 2',5'-trityl-C₃ (compound 45, Table 5) was isolated, purified by DEAE-Sephadex A-25 chromatography and lyophilized to form the amorphous pure compound.

The 5'-monophosphates of the trimer core molecules of the present invention may be prepared from the fully blocked 2',5'-trinucleosidediphosphoditriester by detritylation as in Example 1 followed by reaction with di-p-nitrophenyl-ethylphosphoryl chloride. Extraction, chromatography and deblocking according to Example 1 results in isolation of the 5'-monophosphate trimers. The preparation is exemplified in the method of Example 6.

### Example 6

### Preparation of 5'-0-phosphoryl-3'-deoxyadenylyl (2',5')3'-deoxyadenylyl(2',5')3'-deoxyadenosine

0.1 mmole of N⁶-benzoyl-5'-0-p-methoxytrityl-3'-deoxyadenylyl(2'-o-chlorophenyl-5')N⁶-benzoyl-3'-deoxyadenylyl(2'-o-chlorophenyl-5')N⁶,N⁶,2'-0tribenzoyl-3'-deoxyadenosine is prepared from 3'-deoxyadenosine by benzoylation, 5'-tosylation, and 2'-phosphorylation, with formation of the dinucleoside phosphotriester, N⁶-benzoyl(2-o-chlorophenylphosphoryl-5)3'-deoxyadenosine, by treatment of the reaction products N⁶-benzoyl(2-triethylammonium-o-chlorophenylphosphoryl-5)-5'-tosyl-3'-deoxyadenosine and 2'-cyanoethylphosphoryl-o-chlorophenyl-3'-deoxyadenosine with triisopropylbenzenesulfonyl-nitro-1,2,4-triazolide. The fully blocked dimer thus formed is condensed with N⁶,2'-0-dibenzoyl-3'-deoxyadenosine to form the trimer. 0.01 mM of this trimer prepared according to the method of Charubala, R., and Pfleiderer, W., Tetrahedron Lett. 21, 4077 (1980) was treated with 2 ml of a solution of 2% p-toluenesulfonic acid in dichloromethane/ methanol (7/3, v/v) for 30 minutes at room temperature to remove the p-methoxytrityl group. Purification by silica gel chromatography on a preparative plate with chloroform/methanol (95/5, v/v) gave a 90% yield of the 5'-deprotected analog.

This product was dissolved in 1 ml of absolute pyridine and treated with 0.27 mmole of di--nitrophenylethyl-phosphoryl chloride as described by Himmelsbach, F., and Pfleiderer, W., Tetrahedron Lett. 23, 4973 (1982) for 1 hr. at room temperature. After dilution with 15 ml of chloroform, the reaction mixture was extracted three times with phosphate buffer pH 7. The organic layer was dried over sodium sulfate, filtered, evaporated and coevaporated three times with 10 ml of toluene each time. The residue was purified by silica gel chromatography on preparative plates in chloroform/methanol (9/1, v/v) to yield 81% of 5'-0-di-p-nitrophenylethylphosphoryl-N⁶-benzoyl-3'-deoxyadenylyl-(2'-o-chlorophenyl-5')N⁶-benzoyl-3'-deoxyadenylyl(2'-o-chlorophenyl-5')N⁶,N⁶,2'-0-tribenzoyl-3'-deoxyadenosine in the form of an amorphous solid.

0.01 mmole of the latter material was treated with o-nitrobenzaldoximate according to the deprotection procedure of Example 1 to remove o-chlorophenyl blocking groups. After evaporation to dryness and several coevaporations with absolute pyridine, the deprotected product was dissolved in 10 ml of a 0.5 M solution of diazabicyclo [4.3.0]undecene in absolute pyridine and stirred for 36 hours at room temperature to cleave the p-nitrophenylethyl group by β-elimination. The solution was again evaporated and then treated with 20 ml of concentrated ammonium hydroxide for 24 hours at room temperature. Purification and isolation of the trimer core 5'-monophosphate (compound 46, Table 5) was achieved by DEAE-Sephadex chromatography and lyophilization of the main fraction.

The tetramer core molecules of the present invention may be prepared by following the method of Examples 7 or 8.

### Example 7

### Preparation of adenylyl(2',5')adenylyl(2',5')3'-deoxyadenylyl(2',5')3'-deoxyadenosine

0.5 mmole of fully-protected compound 47 having the formula (VI) and 0.4 mmole of N⁶-benzoyl-3'-deoxyadenylyl (2'-o-chlorophenyl-5')N⁶,2'-0-dibenzoyl-3'-deoxyadenosine (compound 24, Table 3) were dissolved in 5 ml of absolute pyridine.

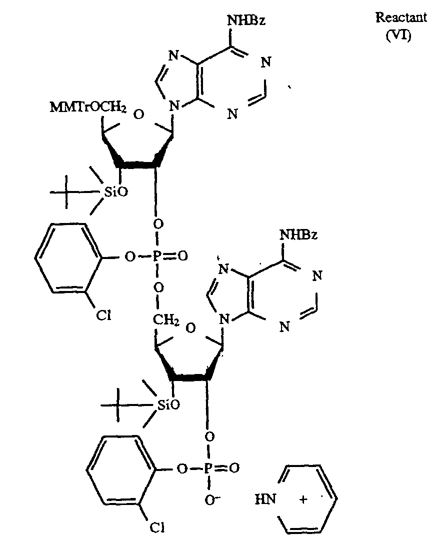

Following addition of 1 mmole of 2,4,6-triisopropylbenzenesulfonyl chloride and 3 mmole of 1-methylimidazone, the mixture was stirred for 2 h at room temperature. The solution was diluted with 400 ml of chloroform, washed twice with 400 ml of water, then the organic layer was dried over sodium sulfate, filtered and evaporated to dryness. The residue was coevaporated twice with 50 ml of toluene. Purification was achieved by chromatography on a silica gel column (20.x 2.5 cm) first with chloroform and then with a gradient of chloroform/methanol of 99/1 to 98/2 (v/v). On evaporation, the main fraction gave compound 48 (Table 6) as a solid foam in 80% yield. Compound 48 is a fully blocked 2',5'-tetranucleosidetriphosphotritriester according to the general formula of tetramer core (VII), below. Deprotection of the blocking groups was performed by the procedure of Example 1 to yield 2',5'-A-A-C-C (compound 49, Table 6). DEAE-Sephadex chromatography, evaporation and lyophilization resulted in an amorphous solid in 80% yield.

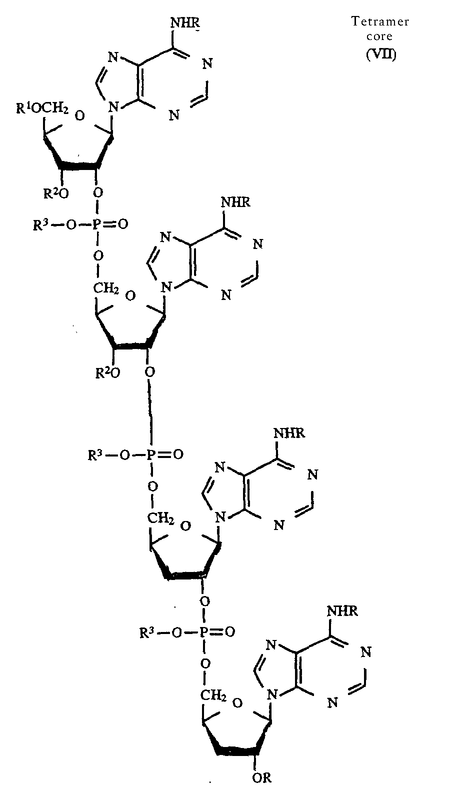

**TABLE 6**

| Compound No. | R | R¹ | R² | R³ |
|---|---|---|---|---|
| 48 | Bz | MMTr | SiTBD | 2-chlorophenyl |
| 49 | H | H | H | H |

### Example 8

### Preparation of 3'-deoxyadenylyl(2',5')3'-deoxyadenylyl(2',5')3'-deoxyadenylyl (2',5')3'-deoxyadenosine

0.1 mmole of N⁶-benzoyl-5'-O-p-methoxytrityl-3'-deoxyadenylyl(2'-o-chlorophenyl-5')N⁶-benzoyl-3'-deoxyadenylyl(2'-o-chlorophenyl-5')N⁶, N⁶, 2'-O-tribenzoyl-3'-deoxyadenosine (compound 50, Table 7), a fully-blocked 2',5'-trinucleosidediphosphoditriester according to the general formula of reactant (VIII),

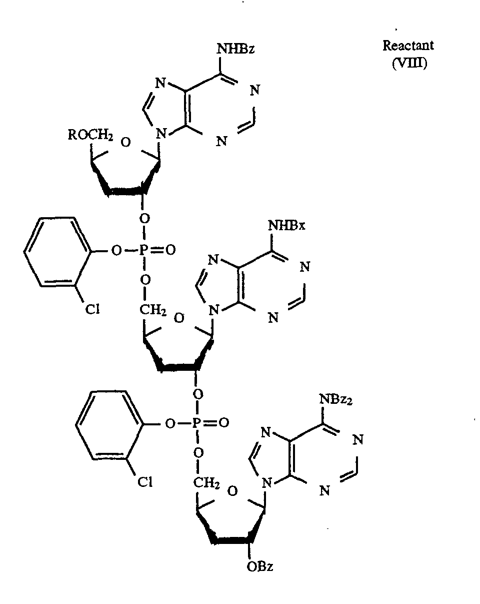

**TABLE 7**

| Compound No. | R |
|---|---|
| 50 | MMTr |
| 51 | H |

was treated with 2 ml of a 2% solution of p-toluenesulfonic acid in dichloromethane/methanol (4/1, v/v) for 30 minutes at room temperature. The reaction was stopped by adding 20 ml of phosphate buffer pH 7. The solution was extracted several times with 200 ml of chloroform. The organic layer was washed with water, dried over sodium sulfate, filtered and evaporated to a small volume for purification on preparative silica gel plates in chloroform/methanol (95/5, v/v). The main band was eluted by chloroform/methanol (4/1, v/v) to give the 5'-detritylated compound 51 (Table 7) upon evaporation in 80% yield.

0.05 mmole of compound 51 (Table 7) was then condensed with 0.1 mmole of pyridinium N⁶-benzoyl-5'-O-p-methoxytrityl-3'-deoxyadenosine-2'-(2-o-chlorophenyl) phosphate (compound 2, Table 1) in 0.6 ml of absolute pyridine in the presence of 0.2 mmole of 2,4,6-triisopropylbenzene-sulfonyl chloride and 0.6 mmole of 1-methylimidazole for 2 h at room temperature. The solution was diluted with 100 ml of chloroform, washed twice with water, dried over sodium sulfate and evaporated to a small volume for separation on preparative silica gel plates in chloroform/methanol (95/5, v/v). The main band was eluted with chloroform to give the fully-protected 2', 5'-tetranucleosidetriphosphotritriester compound 52 (Table 8, below) as an amorphous solid upon evaporation in 84% yield.

The blocking groups of compound 52 were removed according to the procedure of Example 1, followed by DEAE-Sephadex chromatography and lyophilization. Tetramer core 2',5'-C-C-C-C (compound 53, Table 8) resulted as an amorphous solid in 70% yield. The structure of compound 53 is according to the general formula of tetramer core (IX).

The 5'-O-monophosphates of tetramer core molecules may be prepared from the fully blocked 2',5'-tetranucleosidetriphosphotritriester by 5'-detritylation as in Example 1 followed by reaction with di-p-nitrophenylethylphosphoryl chloride. Extraction, chromatography and deblocking according to Example 1 results in isolation of the 5'-O-monophosphate tetramers. The preparation is exemplified in Example 6, above.

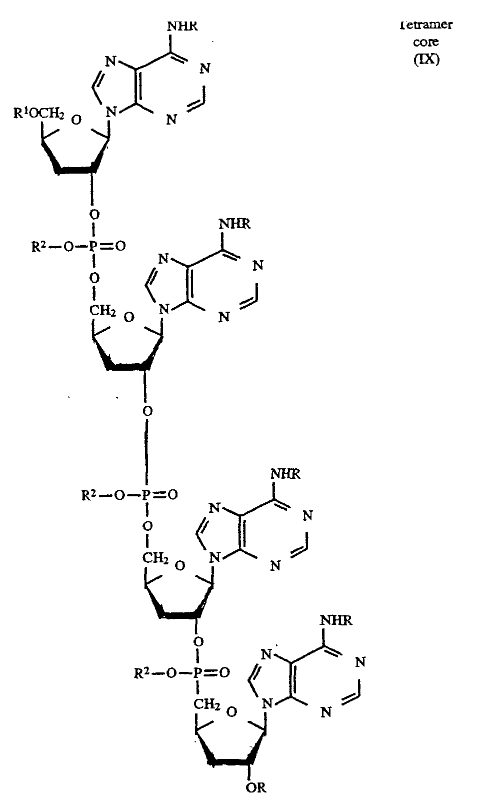

**TABLE 8**

| Compound No. | R | R¹ | R² |
|---|---|---|---|
| 52 | Bz | MMTr | 2-chlorophenyl |
| 53 | H | H | H |

The 5'-diphosphate and 5'-triphosphate of trimer and tetramer core molecules may be prepared by adding 0.5 mM of tributylammonium pyrophosphate dissolved in 5 ml of dimethylformamide to 0.1 mM of monophosphorylated core as the anhydrous tributylammonium salt in 1 ml of dimethylformamide and 0.5 mM of 1,1'-carbonyldiimidazole. After 20 hours at room temperature, the reactants are treated with 5 ml of methanol, evaporated to dryness and chromatographed on a 2 x 20 cm DEAE cellulose column. The 5'-di and triphosphates are isolated following a linear gradient (0-0.4M in 3 1 at pH 7.5) of triethylammoniumbicarbonate. This is the method of Hoard, D.E., and Ott, D.G., J. Amer. Chem. Soc. 87, 1785-1788 (1965). The 5'-diphosphates and 5'-triphosphates may then be purified by DEAE-Sephadex A25 and Sephadex G-10 chromatography.

Structural modification of the 2',5'-oligoadenylate molecule has provided molecules that are potent inhibitors of virus replication, particularly replication of retroviruses, such as HIV. These synthetic molecules are biologically more active and metabolically more stable than the naturally occurring 2',5'-oligoadenylate molecule.

The antiretroviral activity of the compounds of the present invention is demonstrated by the following experimental methods in which the core compound of the invention or its 5' mono, -di-, or triphosphate counterparts may be substituted in the following experiments with the efficacy disclosed for such compounds in the specification.

According to the following experiment, a 5,6-dichlorobenzimidazyl 2',5'-oligoadenylate analog was observed to inhibit HIV-1 reverse transcriptase activity when compared with other 2-5A analogs.

### Example 9

### Inhibition Of HIV-1 Reverse Transcriptase Activity In Vitro

Reverse Transcriptase Assays. Virus was concentrated from cell-free (0.45 micromolar-filtered) conditioned H9/HTLV-III_{B} culture supernatants by centrifugation at 18,000 r.p.m. for 4 h at 20°C in a Beckman JA-20 rotor. A viral pellet obtained from 50 ml of conditioned culture fluid was dissolved in 0.5 mL of a solution containing 17 mM Tris-HCl (pH 7.8), 3mM dithiothreitol (DTT), 55 mM KC1, 0.32% w/v Triton X-100, and 33% glycerol. This viral lysate was stored at -20°C and was used as a source of HIV-1 reverse transcriptase. Reverse transcriptase reactions were performed in 100 µL reaction volumes containing 40 mM Tris-HC1 (pH 7.8), 4 mM DTT, 50 mM KCl, 10 mM MgCl₂, 0.0325% w/v Triton X-100, 3 micromolar [³3H]dTTP (80 Ci/mmol,NEN) and poly (A).(dT)₁₅ (2.5 microgram/mL) template-primer after the addition of 10 microliter enzyme. Inhibitors were added at various concentrations after adjusting water volumes so that reaction volumes remained constant. Reactions were incubated at 37°C for 1 h in a humidified environment and terminated by adding 2 mL of 10% cold trichloroacetic acid. Precipitate was collected on 0.45 µm cellulose-acetate Millipore filters which were then dissolved in 10 mL of 3a70B aqueous scintillant and the counts per minute quantitated using a Beckman LS 6800 liquid scintillation spectrometer.

The effect of a 5,6-dichlorozimidazylyl analog on 2-5A on HIV-1 reverse transcriptase activity is shown in Table 9. The 5,6-dichlorobenzimidazole riboside 2',5'-trimer 2',5'-cordycepin trimer 5'-triphosphate (p₃ C₃) was the most effective inhibitor (73% inhibition at 200 micromolar), followed by 2',5'-pC₄ (64% inhibition at 200 micromolar), the 2'-5'-cordycepin trimer 5'-triphosphate (p₃C₃) (62% inhibition at 200 micromolar), 2'-5'-A-C-A (58% at 200 micromolar) and 2',5'-A-A-ara-A (53% inhibition at 200 micromolar) (Table 9).

**TABLE 9**

| Effect of 2-5A Analogues on HIV-1 Reverse Transcriptase Activity | | |
|---|---|---|
| 2-5A or 2',5' Analogue | Concentration (micromolar) | Percent Inhibition^{a} |
| (adenosine) A | 400 | 0 |
| A₃ | 200 | 0 |
| pA₃ | 200 | 29 |
| p₃ A₃ | 200 | 0 |
| cordycepin (C) | 400 | 0 |
| C₃ | 200 | 31 |
| pC₃ | 200 | 43 |
| p₃ C₃ | 200 | 62 |
| C₄ | 200 | 39 |
| pC₄ | 200 | 64 |
| A-C-C | 200 | 42 |
| A-A-C | 200 | 16 |
| C-A-C | 200 | 36^{b} |
| A-C-A | 200 | 58 |
| A-A-C-C | 200 | 35^{b} |
| A-A-C-A | 200 | 7 |
| pI₃ | 200 | 35 |
| A-A-ara-A | 200 | 53 |
| Tu-Tu-Tu | 200 | 41 |
| xylo-A₄ | 200 | 15 |
| A-A-A-3'-amino | 200 | 43 |
| EHNA-A-A | 200 | 36 |
| 5,6-dichlorobenzimidazylyl(2'-5')-5,6-di-chlorobenzimidazylyl-(2',5')-5,6-dichlorobenzimidazole riboside | 200 | 73 |

| | | |
|---|---|---|
| ^{a} Values for control reactions (i.e. no inhibitor present) were greater than 180,000 cpm while blank reaction (no enzyme present) values were less than 12,000 cpm. | | |
| ^{b} Average of two or more experiments. | | |

## Claims

1. 5,6-Dichlorobenzimidazylyl(2',5') 5,6-dichlorobenzimidazylyl(2',5') 5,6-dichlorobenzimidazole riboside, the 5' mono-, di-, and triphosphates thereof, and pharmaceutically acceptable salts of any of them.

2. A compound according to claim 1 for use in medicine.

3. A pharmaceutical composition comprising a compound according to claim 1 and a pharmaceutically acceptable carrier.

4. Use of a compound according to claim 1 for the preparation of a medicament for treating retroviral infections.

## Patentansprüche

1. 5,6-Dichlorbenzimidazylyl (2', 5') 5,6-dichlorbenzimidazylyl (2', 5') 5,6-dichlorbenzimidazolribosid, das 5' Mono-, Di- und Triphosphat von diesem und pharmazeutisch verträgliche Salze von jedem derselben.

2. Verbindung nach Anspruch 1 zur Verwendung in der Medizin.

3. Pharmazeutische Zusammensetzung enthaltend eine Verbindung nach Anspruch 1 und einen pharmazeutisch verträglichen Träger.

4. Verwendung einer Verbindung nach Anspruch 1 zur Herstellung eines Arzneimittels zum Behandeln von Retrovirus-Infektionen.

## Revendications

1. 5,6-dichlorobenzimidazylyl(2',5') 5,6-dichlorobenzimi-dazylyl(2',5') 5,6-dichlorobenzimidazole riboside, leurs 5' mono-, di- et triphosphates, et les sels pharmaceutiquement acceptables de chacun de ceux-ci.

2. Composé selon la revendication 1 pour l'utilisation en médecine.

3. Composition pharmaceutique comprenant un composé selon la revendication 1 et un excipient pharmaceutiquement acceptable.

4. Utilisation d'un composé selon la revendication 1 pour la préparation d'un médicament pour traiter les infections rétrovirales.
